# EUROPEAN PATENT APPLICATION

(11) **EP 4 046 683 A1**
(43) Date of publication of application: **24.08.2022**
(21) Application number: 19948987.3
(22) Date of filing: 08.11.2019
(51) Int. Cl.: A61N 5/06

(54) **MOUTHPIECE-TYPE TREATMENT DEVICE**

(30) Priority: 17.10.2019 KR 20190129164
(71) Applicant: Welsmeditech Co., Ltd., Chungcheongnam-do 31035 (KR)
(72) Inventor: HWANG, Kyong Won, Seoul 01197 (KR); MOON, Pyoung Su, Cheonan-si, Chungcheongnam-do 31101 (KR)
(74) Representative: Gulde & Partner
(86) International application number: PCT/KR2019/015206
(87) International publication number: WO 2021/075626

(57) **Abstract**

The present invention relates to a mouthpiece-type treatment device which is capable of treating inflammation in the oral cavity such as in the gums by having a light irradiating means inside the mouthpiece, and which has a plurality of modules that can be detached from the mouthpiece so as to be usable by both children and adults regardless of the size of the oral cavity, the treatment device comprising: a main body comprising a mouthpiece which is inserted into the oral cavity, and a connection part; a cell regeneration lamp provided inside the mouthpiece; a control part for controlling the operation of the cell regeneration lamp; and a plurality of coupling modules which are coupled to the respective ends of the mouthpiece, wherein the cell regeneration lamp can output near-infrared rays, can be used by all patients of various oral cavity sizes, and can treat stomatitis occurring not only on the gums but also the palate, the membrane lining the cheek, etc.

## Description

### [Technical Field]

The present invention relates to a mouthpiece-type treatment device, and more particularly, to a mouthpiece-type treatment device in which a light irradiation unit is provided inside a mouthpiece to treat inflammation in an oral cavity such as in the gums and a coupling module may be detachably attached to the mouthpiece so that both children and adults may use the mouthpiece-type treatment device regardless of the size of the oral cavity.

### [Background Art]

Inflammatory diseases that occur in the tongue, the gums, the roof of the mouth, the buccal mucosa, or the like are generally referred to as stomatitis. In general, when mild stomatitis occurs, a drug is directly applied to the oral cavity, and when severe stomatitis occurs, the drug is directly applied to the oral cavity, and at the same time, an internal medicine is administered.

In the related art, when a drug is directly applied to the oral cavity, the drug is quickly washed away by saliva, a treatment effect does not last, and thus a treatment period becomes longer. Further, it is difficult to prescribe the internal medicine to children, some patients, and pregnant women.

In order to solve this problem, a periodontal disease phototherapy device including a mouthpiece member having a fitting bone that allows the mouthpiece member to be inserted into the mouth to cover parts of the teeth and gums, a light source array in which a plurality of light sources are arrayed in the mouthpiece member to irradiate the teeth and the gums with light, and a controller that controls driving of the light sources has been developed, and such a mouthpiece-type periodontal disease phototherapy device is disclosed in detail in Korean Patent No. 10-0777438 (registered on November 12, 2007).

However, in the case of the related art including the above document, since the size of the mouthpiece into which the light sources are inserted is fixed, it is still difficult for patients having various oral sizes, such as children and adults, to use the periodontal disease phototherapy device.

Further, in the case of the related art including the above document, when stomatitis occurs on the roof of the mouth and the buccal mucosa in addition to the teeth and the gums, light irradiation is difficult.

Further, in the case of the related art including the above document, although the mouthpiece in which the controller and the light source array are installed is an electronic device connected by a driving wire, it is difficult to prevent malfunctions caused by inflow of an oral fluid into the device.

### [Disclosure]

### [Technical Problem]

The present invention is directed to providing a mouthpiece-type treatment device in which a coupling module is detachably attached to a mouthpiece provided with a cell regeneration lamp so that all patients having various sizes of oral cavities may use the treatment device, inflammation occurring in the roof of the mouth and the buccal mucosa in addition to the gums may be treated, and malfunction of electronic devices due to an oral fluid is prevented.

### [Technical Solution]

One aspect of the present invention provides a mouthpiece-type treatment device including a body including a mouthpiece to be inserted into an oral cavity and a connection part, a cell regeneration lamp provided inside the mouthpiece, a controller that controls operation of the cell regeneration lamp, and a plurality of coupling modules coupled to both ends of the mouthpiece, wherein the cell regeneration lamp outputs near-infrared light.

Coupling grooves may be formed in both ends of the mouthpiece, the coupling modules may each include a light conveyor embedded in and protruding from one end thereof, and the light conveyor may be coupled to the coupling groove.

A transfer lamp may be embedded in the coupling groove, the light conveyor may transfer light radiated from the transfer lamp, and the controller may control light output intensities and output times of the cell regeneration lamp and the transfer lamp.

Each of the mouthpiece and the coupling module may include an upper part into which upper teeth and upper gums are inserted and a lower part into which lower teeth and lower gums are inserted, the upper part may include a first flat portion occluded with the upper teeth and a first side portion facing side surfaces of the upper teeth and the upper gums, and the lower part may include a second flat portion occluded with the lower teeth and a second side portion facing side surfaces of the lower teeth and the lower gums.

The cell regeneration lamp may irradiate inner and outer side surfaces of the gums from the first side portion and the second side portion with light, and a plurality of light guide grooves may be formed in an outer side of the mouthpiece.

### [Advantageous Effects]

A mouthpiece-type treatment device according to the present invention can be used for patients having various sizes of oral cavities, such as children and adults, as a plurality of coupling modules having a light conveyor installed therein are attached to and detached from a mouthpiece having a cell regeneration lamp installed therein.

Further, in the mouthpiece-type treatment device according to the present invention, a plurality of light guide grooves are formed in an outer side of the mouthpiece and the coupling module, light output from the cell regeneration lamp and the light conveyor installed therein can be effectively emitted, and thus stomatitis occurring in the roof of the mouth and the buccal mucosa in addition to the gums can be treated.

Further, in the mouthpiece-type treatment device according to the present invention, an oral fluid prevention part is formed on one side of a terminal, and thus an oral fluid of a patient wearing the mouthpiece can be prevented from flowing into a body or a controller.

### [Description of Drawings]

FIG. 1 is an exploded perspective view of a mouthpiece-type treatment device according to an embodiment of the present invention.
FIG. 2 is a side cross-sectional view of the mouthpiece-type treatment device according to the embodiment of the present invention.
FIG. 3 is an enlarged view of the side cross-sectional view of the mouthpiece-type treatment device according to the embodiment of the present invention.
FIG. 4 is an exemplary view of a use state of the mouthpiece according to an embodiment of the present invention.
FIG. 5 is an exemplary view of a use state of a coupling module according to an embodiment of the present invention.

### [Modes of the Invention]

Hereinafter, exemplary embodiments of the present invention will be described in detail with reference to the accompanying drawings.

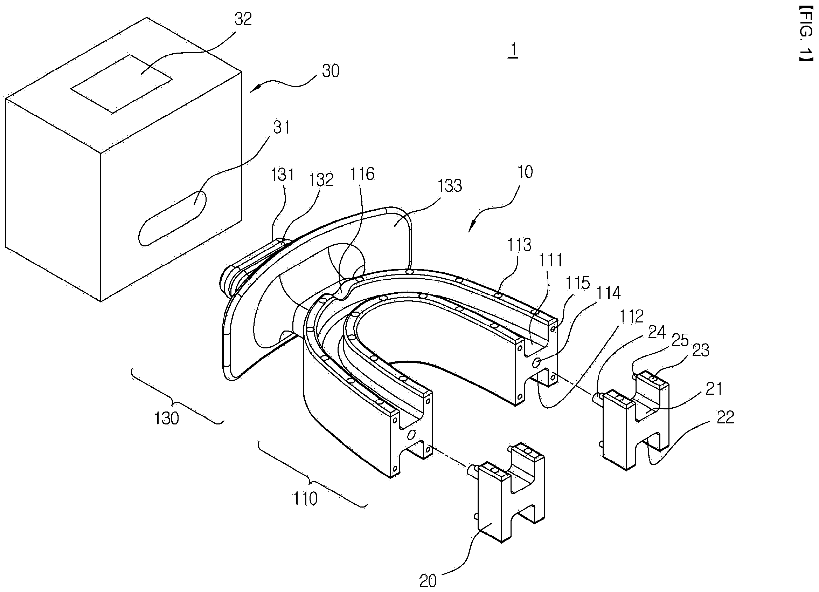

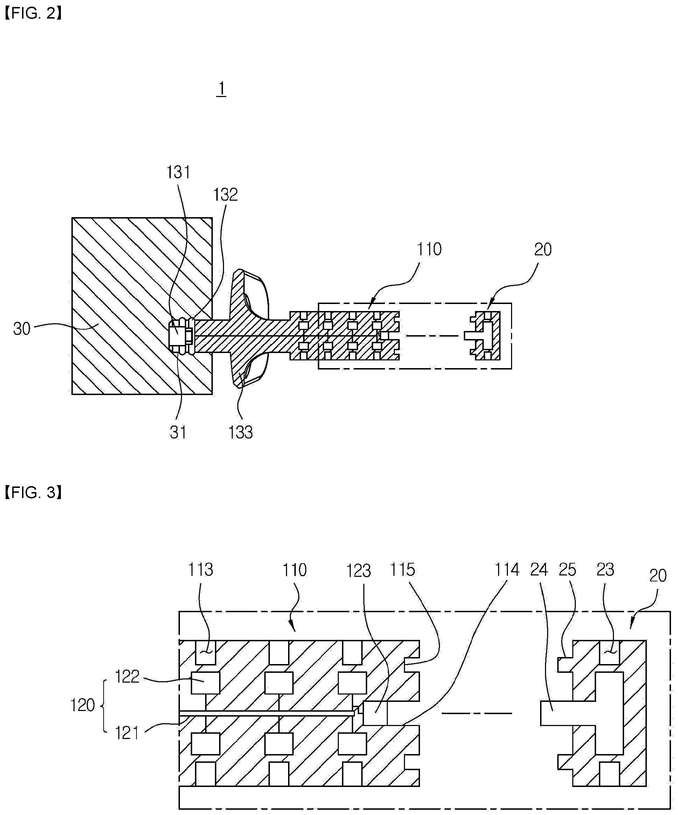

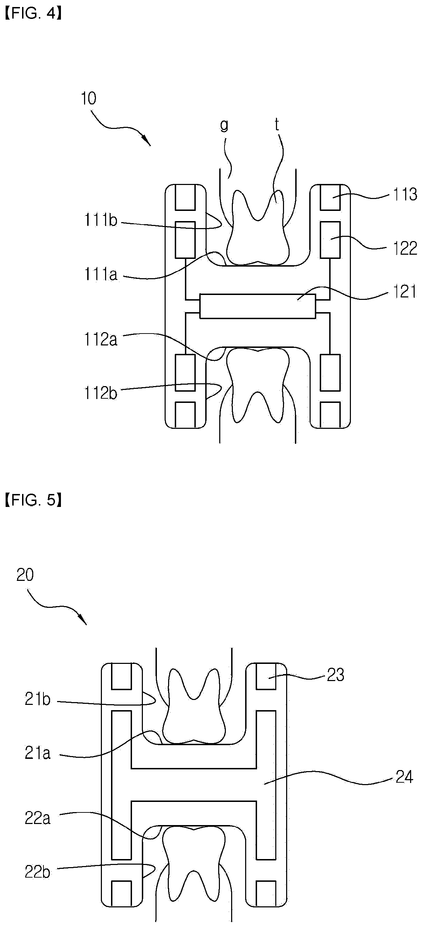

FIG. 1 is an exploded perspective view of a mouthpiece-type treatment device according to an embodiment of the present invention, FIG. 2 is a side cross-sectional view of the mouthpiece-type treatment device according to the embodiment of the present invention, FIG. 3 is an enlarged view of the side cross-sectional view of the mouthpiece-type treatment device according to the embodiment of the present invention, FIG. 4 is an exemplary view of a use state of the mouthpiece according to an embodiment of the present invention, and FIG. 5 is an exemplary view of a use state of a coupling module according to an embodiment of the present invention.

A mouthpiece-type treatment device 1 according to the present invention may include a body 10, a plurality of coupling modules 20 coupled to both ends of the body 10, and a controller 30 coupled to one side of the body 10.

The body 10 includes a mouthpiece 110 to be inserted into an oral cavity, a substrate part 120 formed to radiate light from the inside of the mouthpiece 110, and a connection part 130 that electrically connects the substrate part 120 to the outside, and the outer shape of the body 10 is formed using light-transmitting silicone by molding. The method for manufacturing the body 10 is not limited thereto, and any manufacturing process may be applied as long as the substrate part 120 may be inserted into and installed inside the body 10.

As illustrated in FIGS. 4 and 5, the mouthpiece 110 has a U shape corresponding to arrangement of the teeth t and gums g of a patient and includes a first upper part 111 having tooth grooves formed in an upper side thereof so that the upper teeth and upper gums may be occluded together and a first lower part 112 having tooth grooves formed in a lower side thereof so that the lower teeth and lower gums may be occluded together.

The first upper part 111 has a first flat portion 111a occluded with the upper teeth and a first side portion 111b facing side surfaces of the upper teeth and the upper gums.

Further, the second lower part 112 has a second flat portion 112a occluded with the lower teeth and a second side portion 112b facing side surfaces of the lower teeth and the lower gums.

As illustrated in FIGS. 2 and 3, the substrate part 120 includes a substrate 121 and a cell regeneration lamp 122.

The substrate 121 is made of the same flexible material as the mouthpiece 110 and is formed in a U shape, and the substrate 121 is embedded in an intermediate flat portion between the first flat portion 111a and the second flat portion 112a.

The cell regeneration lamp 122 connected to the substrate 121 is embedded in the first side portion 111b and the second side portion 112b to correspond to locations of the teeth and the gums. The cell regeneration lamp 122 is formed as a light emitting diode (LED) or a laser light source that outputs light having a near-infrared wavelength of 675 nm to 808 nm and may allow the light to pass through the silicone forming the mouthpiece 110 to be radiated to the outside. The cell regeneration lamp 112 may output the near-infrared light and thus may increase cellular energy by activating an adenosine triphosphate reaction of cells in an inflammation site. Due to the increased cellular energy, cell regeneration is activated, and thus stomatitis may be effectively treated. In the above description, a near-infrared lamp is used as the cell regeneration lamp 122, but the present invention is not limited thereto, and any light source that is effective for cell regeneration, such as an ultraviolet lamp that may activate cell regeneration according to sterilization of harmful bacteria in the inflammation by irradiation with ultraviolet light, may also be applied.

A coupling groove 114 and an indication groove 115 are formed in both ends of the U shape of the mouthpiece 110, and thus the mouthpiece 100 may be coupled to the coupling modules 20.

The coupling groove 114 is a groove formed between the first flat portion 111a and the second flat portion 112a.

As illustrated in FIG. 3, a transfer lamp 123 that is the same as the cell regeneration lamp is embedded in the coupling groove 114.

The indication groove 115 is provided as a plurality of grooves formed in upper and lower sides of the coupling groove 114.

A superior labial frenulum escape groove 116 is a groove formed such that the superior labial frenulum may be seated thereon when the mouthpiece 110 is worn.

A light conveyor 24 may protrude from and be embedded in a central part of the coupling module 20. A protrusion portion of the light conveyor 24 is inserted into the coupling groove 114, and the light conveyor 24 is made of a light guide member such as polymethyl methacrylate (PMMA) resin or has an optical fiber cable embedded therein and thus may transfer the light output from the cell regeneration lamp 122 provided in the coupling groove 114.

The coupling module 20 may be made of a light-transmitting silicone material as in the mouthpiece 110 so that the light radiated from the light conveyor 24 may be emitted to the outside of the coupling module 20.

As illustrated in FIG. 1, the coupling module 20 includes a second upper part 21 having tooth grooves formed in an upper side thereof so that the upper teeth and the upper gums may be occluded together and a second lower part 22 having tooth grooves formed in a lower side thereof so that the lower teeth and the lower gums may be occluded together.

The second upper part 21 has a third flat portion 21a occluded with the upper teeth and a third side portion 21b facing side surfaces of the upper teeth and the upper gums.

The second lower part 22 has a fourth flat portion 22a occluded with the lower teeth and a fourth side portion 22b facing side surfaces of the lower teeth and the lower gums.

As illustrated in FIG. 5, the light conveyor 24 has an H shape so that the third side portion 21b and the fourth side portion 22b may be irradiated with light.

A plurality of indication bosses 25 are provided on one end of the coupling module 20, the indication bosses 25 are inserted into the indication grooves 115, and thus the coupling module 20 coupled to the mouthpiece 110 is coupled without inclination. Due to the above configuration, the teeth of the patient may be stably occluded with the mouthpiece 110 and the coupling module 20.

The plurality of indication bosses 25 are protrusions made of a silicone material, but when a large amount of light irradiation required for treatment of the patient is required, the indication bosses 25 may be formed of the same light guide member as the light conveyor 24.

In the present invention having the above configuration, while the plurality of coupling modules 20 may be attached to or detached from the mouthpiece 110, the coupling modules 20 may also be irradiated with the light, and thus the present invention can be used for patients having various sizes of oral cavities.

Further, a plurality of light guide grooves 113 are formed in an outer side of the mouthpiece 110, and thus the roof of the mouth and the buccal mucosa in addition to the gums may be irradiated with the light.

A plurality of light guide grooves 23 are formed in an outer side of the coupling module 20, and thus when the coupling module 20 is coupled to the mouthpiece 110, the roof of the mouth and the buccal mucosa in addition to the gums can be irradiated with the light.

With the present invention having the above configuration, stomatitis occurring in the roof of the mouth, the buccal mucosa, or the like in addition to inflammation of the gums can be treated.

As illustrated in FIG. 1, the connection part 130 includes a terminal 131, a separation prevention part 132, and an oral fluid prevention part 133.

The terminal 131 is electrically connected to the substrate 121. The terminal 131 is connected to an inner side of a terminal port 31 of the controller 30, and a light output intensity, an output time, and a wavelength of the cell regeneration lamp 122 may be adjusted by operating a control panel 32.

The separation prevention part 132 doubly protruding to increase a contact force with the terminal port 31 is formed outside the terminal 131. Due the separation prevention part 132, a fastening force may be maintained even with slight movement of the patient undergoing light treatment.

Since it is difficult for the patient wearing the mouthpiece 110 to smoothly swallow an oral fluid, when the oral fluid flows to the outside of the mouthpiece to flow into the terminal 131 or into the terminal port 31, the mouthpiece-type treatment device 1 may malfunction due to a short circuit. To prevent this, the oral fluid prevention part 133 having a rectangular protruding shape is provided on one side of the terminal 131.

## Claims

1. A mouthpiece-type treatment device comprising:
a body including a mouthpiece to be inserted into an oral cavity and a connection part;
a cell regeneration lamp provided inside the mouthpiece;
a controller that controls operation of the cell regeneration lamp; and
a plurality of coupling modules coupled to both ends of the mouthpiece,
wherein the cell regeneration lamp outputs near-infrared light.

2. The mouthpiece-type treatment device of claim 1, wherein coupling grooves are formed in both ends of the mouthpiece,
the coupling modules each include a light conveyor embedded in and protruding from one end thereof, and
the light conveyor is coupled to the coupling groove.

3. The mouthpiece-type treatment device of claim 2, wherein a transfer lamp is embedded in the coupling groove,
the light conveyor transfers light radiated from the transfer lamp, and
the controller controls light output intensities and output times of the cell regeneration lamp and the transfer lamp.

4. The mouthpiece-type treatment device of claim 1, wherein each of the mouthpiece and the coupling module includes an upper part into which upper teeth and upper gums are inserted and a lower part into which lower teeth and lower gums are inserted,
the upper part includes a first flat portion occluded with the upper teeth and a first side portion facing side surfaces of the upper teeth and the upper gums, and
the lower part includes a second flat portion occluded with the lower teeth and a second side portion facing side surfaces of the lower teeth and the lower gums.

5. The mouthpiece-type treatment device of claim 4, wherein the cell regeneration lamp irradiates inner and outer side surfaces of the gums from the first side portion and the second side portion with light, and
a plurality of light guide grooves are formed in an outer side of the mouthpiece.
